Europäisches Patentamt

European Patent Office

Office européen des brevets

(19)

(11) Numéro de publication: **0 246 129**
**A1**

(12) # DEMANDE DE BREVET EUROPEEN

(21) Numéro de dépôt: 87400909.5

(22) Date de dépôt: 17.04.87

(51) Int. Cl.³: **G 01 N 9/32**
**A 61 M 16/01**

Une requête en recification de la description (page 3) a été présentée conformément à la règle 88 CBE. Il est statué sur cette requête au cours de la procédure engagée devant la division d'examen.

(30) Priorité: 22.04.86 FR 8605988

(43) Date de publication de la demande:
19.11.87 Bulletin 87/47

(84) Etats contractants désignés:
AT BE CH DE ES GB GR IT LI LU NL SE

(71) Demandeur: Corfa, Yves-Paul
La Briantière Oudon
F-44150 Ancenis(FR)

(72) Inventeur: Corfa, Yves-Paul
La Briantière Oudon
F-44150 Ancenis(FR)

(74) Mandataire: Lemonnier, André
4 Boulevard Saint-Denis
F-75010 Paris(FR)

(54) **Dispositif de sécurité contre l'Inversion des branchements dans les appareillages à mélange de gaz.**

(57) La présente invention a pour objet un dispositif de sécurité contre l'inversion des branchements dans les appareillages à mélange de gaz.

Le dispositif comporte, pour chaque gaz alimenté, un dispositif (10,11) de commutation à retard sensible à la densité du gaz réellement alimenté, l'ensemble des dispositifs de commutation contrôlant, dans l'ordre décroissant desdites densités, l'ordre dans lequel se produit une variation de pression dans les différentes canalisations (28-29) de liaison reliant ces dispositifs de commutation à une vanne de commutation (12) qui est ouverte pour assurer les intercommunications sur toutes les canalisations (14-15-16-17) de l'appareil de mélange entre l'alimentation (1-2) et l'utilisation lorsque, et seulement lorsque, la canalisation de liaison correspondant au gaz théoriquement le plus léger est soumise la première à ladite variation de pression, ladite ouverture de la vanne interrompant l'alimentation, à partir d'au moins une (14) des canalisations d'alimentation en gaz de l'appareil de mélange, d'un détecteur de pression (18) commandant une alarme.

L'invention trouve son application dans les appareillages d'anesthésie et de respiration assistée.

<u>Dispositif de sécurité contre l'inversion des branchements dans</u>
<u>les appareillages à mélange de gaz.</u>

La présente invention concerne les appareillages assurant un mélange de deux ou plusieurs gaz, notamment mais non exclusivement
les équipements médicaux d'anesthésie-réanimation qui alimentent
les appareils respiratoires ou d'anesthésie avec un mélange d'oxygène et de protoxyde d'azote.

Dans ces appareils, l'entrée des gaz se fait par l'intermédiaire
de raccords de sécurité qui sont rendus spécifiques d'un gaz donné par des formes complémentaires partie mâle-partie femelle du
raccord, formes qui diffèrent selon les gaz. Toutefois, la partie amovible de chaque raccord est raccordée à l'appareillage par un tuyau souple fixé sur les canules par des colliers de serrage et il est toujours possible que les parties amovibles des raccords caractérisées par leurs formes complémentaires des parties fixes soient interverties, ce qui inverse la nature des gaz alimentés à l'appareil de mélange. La même erreur peut se produire lors des changements des bouteilles de la centrale ou lors des travaux effectués sur les canalisations de distribution. De telles inversions modifient les taux de mélange des gaz et les conséquences peuvent être d'une extrême gravité.

Certains équipements connus utilisent comme sécurité des procédés de reconnaissance des gaz mettant en oeuvre des appareils faisant appel à une énergie externe, en général électrique, ce qui impose des sujétions de mise en oeuvre et ces appareils d'analyse sont onéreux et fragiles de sorte que la sécurité est très souvent limitée à des appareils détectant l'absence d'alimentation en l'un des gaz du mélange, notamment en protoxyde d'azote, comme décrit dans GB-A-2.001.249 par exemple.

La présente invention a pour but de réaliser un dispositif de sécurité incorporé à l'appareil respiratoire qui soit d'une conception et d'un fonctionnement simples et sûrs et qui fonctionne en utilisant une pression gazeuse de quelques centaines de kPa qui est la pression d'alimentation des gaz dans les salles d'opération et de réanimation.

La présente invention a pour objet un dispositif de sécurité contre l'inversion des branchements dans les appareillages à mélan- nue à retentir, la prolongation dans le temps du signal d'alarme indiquant que l'appareillage ne peut être utilisé par suite d'une inversion des densités des gaz alimentés.

Dans le cas d'un appareillage mélangeant plus de deux gaz, on peut monter en série un nombre de vannes de commutation égal au nombre des gaz mélangés moins un, ces vannes étant commandées à l'ouverture, de l'amont vers l'aval, par la variation de pression dans la canalisation de liaison correspondant théoriquement au gaz ayant la densité la plus faible ou au gaz dont la densité est, dans l'échelle des densités des gaz mélangés, immédiatement supérieure à celle du gaz de commande de la vanne immédiatement en amont.

Selon une autre caractéristique, le dispositif de commutation à retard sensible à la densité du gaz réellement alimenté est le dispositif connu dit "minuterie pneumatique" dans lequel le gaz sous pression est introduit, par un ajutage d'étranglement, dans un volume, la pression dans ce volume étant appliquée comme signal à une vanne tout ou rien qui interrompt l'envoi de la pression d'alimentation à la canalisation de liaison qu'elle contrôle lorsque ladite pression signal atteint une valeur de seuil. On pourrait également utiliser une vanne de ce type qui ouvre le circuit entre l'alimentation et la canalisation de liaison.

Selon une autre caractéristique, le dispositif de sécurité comporte, en amont des dispositifs de commutation à retard et sur les canalisations les alimentant en gaz sous pression, un dispositif d'égalisation des pressions et de synchronisation de l'envoi des pressions égalisées aux dispositifs de commutation à retard. Ce dispositif est de préférence constitué par un détendeur pilote et au moins deux détendeurs à contre-pression, le premier détendeur à contre-pression étant piloté par la pression en sortie du détendeur pilote et le second détendeur à contre pression et les suivants étant pilotés par la pression à la sortie du détendeur à contre-pression d'ordre immédiatement moins élevé. Cette caractéristique supprime toute incidence sur le fonctionnement de l'ordre dans lequel sont réalisés les branchements des canalisations lorsque le détendeur pilote et le dernier détendeur à contre-pression sont alimentés à partir de la même canalisation.

Selon un mode de réalisation préférentiel dans le cas de l'alimentation avec deux gaz, la vanne de commutation est une vanne à tiroir à double commande pneumatique avec sollicitation à la fermeture par un organe de rappel élastique et les canalisations d'arrivée des gaz depuis les dispositifs de commutation qui aboutissent aux cylindres de commande de la vanne à tiroir sont indépendantes des canalisations d'alimentation en gaz de l'appareillage mélangeur, canalisations dont les intercommunications sont contrôlées par la vanne.

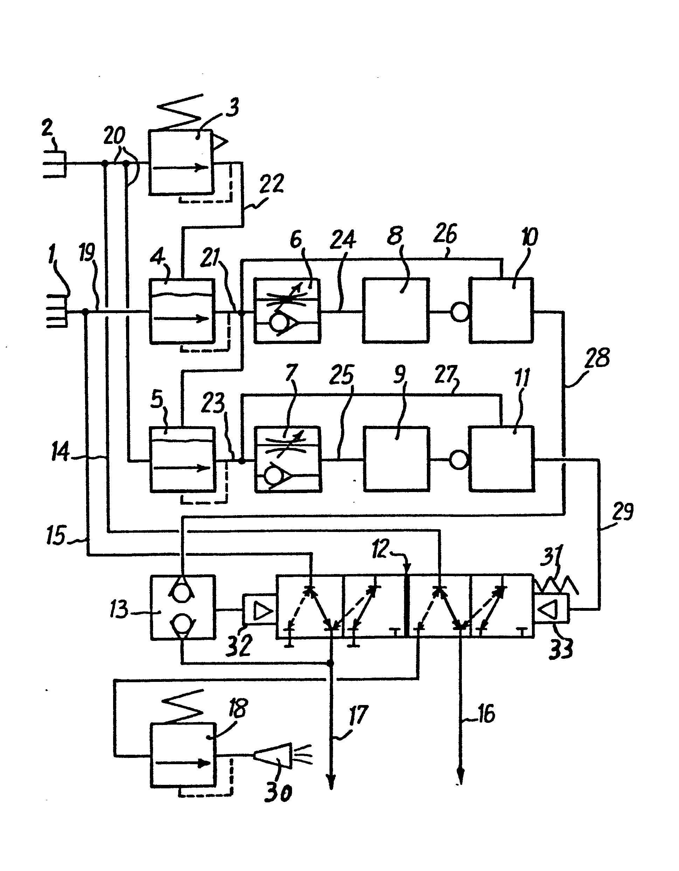

L'invention sera décrite plus en détail ci-après sous forme d'un mode de réalisation avec référence au dessin ci-annexé qui représente schématiquement un dispositif de sécurité conforme à l'invention dans le cas d'un appareillage de mélange de deux gaz.

Dans le mode de réalisation représenté deux raccords d'entrée, un raccord 1 à quatre crans pour le protoxyde d'azote et un raccord 2 à trois crans pour l'oxygène, sont fixés de façon indémontable sur le corps de l'appareil de mélange des gaz et au besoin plombés, de façon que l'inversion des canalisations ne puisse se produire à leur niveau ou en aval. Le raccord 1 est relié par la conduite 19 à un détendeur 4 et le raccord 2 est relié par la conduite 20 aux détendeurs 3 et 5. Le détendeur 3 est équilibré par un ressort au réglage fixe, les détendeurs 4 et 5 sont des détendeurs de recopie d'une pression pilote dans lesquels le ressort est remplacé par la pression à recopier agissant sur la membrane. Le détendeur 3 alimente par la conduite 22 la chambre à membrane du détendeur 4 et le détendeur 4 alimente par la canalisation 21 la chambre à membrane du détendeur 5, les détendeurs 3 et 5 étant alimentés à partir du même raccord, ici le raccord 2 correspondant à l'oxygène. Quel que soit le raccord branché le premier, les débits sortant des détendeurs 4 et 5 apparaîtront quasi-simultanément et les pressions en sortie seront pratiquement égales. En effet, le détendeur 4 ne débitera que si le détendeur 3 l'alimente et si la conduite 19 est sous pression, et le détendeur 5 ne débitera que si le détendeur 4 l'alimente et si la conduite 20 est également sous pression. On aura donc, au moins, des écarts dans les pressions et dans les temps de départ constants, ces écarts étant dus aux réglages et au délai de fonctionnement des détendeurs à contre-pression 4 et 5.

Les détendeurs 4 et 5 alimentent, respectivement par les conduites 21 et 23, des ajutages d'étranglement 6 et 7. Ces ajutages d'étranglement sont du modèle comportant un clapet anti-retour et sont classiques. Les ajutages d'étranglement 6 et 7 alimentent respectivement, par les conduites 24 et 25, les capacités 8 et 9 et les entrées "signal" des vannes pilotées dite "NON A SEUIL" 10 et 11. L'entrée "pression" desdites vannes est alimentée avec la pression à la sortie des détendeurs 4 et 5 par les conduites 26 et 27 branchées respectivement sur les conduites 21 et 23.

Ces deux vannes 10 et 11 sont d'un type classique dans lequel la commutation de la vanne interrompant la communication entre les canalisations 26 et 27 et respectivement 28 ou 29, est réalisée lorsque la pression venant de l'entrée "signal" atteint une certaine proportion de celle appliquée à l'entrée "pression".

Le raccord 1 est alimenté en protoxyde d'azote qui est plus dense que l'oxygène puisque leurs masses moléculaires sont de 44 et de 32, et si le raccord 2 est alimenté en oxygène, la vanne 10 débitera plus longtemps que la vanne 11 puisque, pour le protoxyde d'azote de plus grande densité, la vitesse de fuite par l'ajutage d'étranglement 6 sera plus faible que la vitesse de fuite de l'oxygène par l'orifice d'étranglement 7. De ce fait la pression dans la capacité 8 montera plus lentement que dans la capacité 9. S'il y a inversion à l'entrée, ce sera le contraire et la vanne 11 débitera plus longtemps que la vanne 10.

La vanne 10 alimente par une conduite 28 le cylindre de commande 32 d'une vanne 12 à travers un sélecteur de circuit 13 et la vanne 11 alimente par la conduite 29 l'autre cylindre de commande 33 de la même vanne 12. Cette vanne 12 est formée de deux vannes 3/2 classiques accouplées. Ses deux extrémités sont équipées chacune d'un même cylindre de commande pneumatique 32-33 mais l'extrémité alimentée par la conduite 29 est, en plus, munie d'un ressort 31. De la sorte, la vanne à tiroir 12 conserve la même position que ses deux vérins d'extrémité 32-33 soient alimentés simultanément ou qu'ils n'y soient pas du tout. Par contre, elle bascule si le vérin 32 alimenté par la conduite 28 est alimenté seul. D'autre part, la pression est amenée à la vanne 12 sur son circuit normal par la conduite 14 venant du raccord 2 et par la conduite 15 venant du raccord 1. Dans la position où la rappelle son ressort 31, la vanne 12 assure les connexions représentées en tiretés: le passage à travers la vanne 12 de la conduite 15 vers la sortie 16 est obturé, par contre le flux venant de la conduite 14 est dirigé dans cette position vers un dispositif d'alarme sonore formé d'un détendeur 18 et d'un sifflet 30. La conduite 14 voit elle aussi dans la même position la communication vers la sortie 17 interrompue et toute la partie alimentée par les conduites 16 et 17 est alors à l'échappement. Une dérivation de la conduite 16 alimente l'autre entrée du sélecteur de circuit 13.

Si les raccords sont correctement alimentés, c'est-à-dire le raccord 2 en oxygène et le raccord 1 en protoxyde d'azote, la pression montera plus lentement dans la capacité 8 que dans la capacité 9 et la vanne 10 demeurera ouverte plus longtemps que 11. De ce fait, le côté de la vanne 12 ne comportant pas le ressort 31 sera alimenté plus longtemps que l'autre et lorsque la vanne 11 commute en coupant l'envoi de la pression de la canalisation 27

**0246129**

sur la canalisation 29, la vanne basculera la force engendrée par la pression dans le sélecteur de circuit 13 étant supérieure à la force du ressort 31. A ce moment les interconnexions représentées en traits pleins sont établies par la vanne 12 et la dérivation de la conduite 16 alimente le sélecteur de circuit 13, ce qui maintient la vanne 12 dans la position ainsi acquise et ce, tant que les pressions demeureront sans changement. De même la conduite 17 sera alimentée et l'alarme sonore 18, 30 ne sera plus alimentée.

Si l'on déconnecte les entrées de gaz, la vanne 12 revient dans la position initiale sous l'action du ressort 31 et le cycle de contrôle recommencera à la prochaine connexion.

Si, par contre, les entrées sont inversées, c'est-à-dire si l'oxygène est raccordé à l'entrée 1 et le protoxyde d'azote à l'entrée 2, c'est la vanne 11 qui alimentera le plus longtemps la canalisation 29 et la vanne 12 demeurera dans sa position initiale malgré la chute de pression dans la canalisation 29. Les circuits vers les conduites 16 et 17 ne seront jamais établis et l'alarme sonore 30 continuera à retentir.

Ce cycle se déroulera apparemment de la façon suivante : branchement des deux entrées; signal sonore pendant le temps de fonctionnement des dispositifs de commutation (10 à 12 s), puis arrêt du signal sonore 30 et passage des gaz.

D'autres aménagements de ces circuits peuvent être utilisés, par exemple on peut aménager les ajutages d'étranglement 6 et 7 et les vannes 10 et 11 pour que le cycle se reproduise périodiquement, ce qui permet de détecter une modification de la nature des gaz se produisant après le raccordement et due par exemple au mauvais fonctionnement d'un mélangeur dans une salle d'anesthésie voisine. On peut aussi disposer différemment les vannes de sécurité, utiliser un autre moyen de signalisation, ou encore utiliser des capacités 8 et 9 variables avec les ajutages d'étranglement 6 et 7 fixes. On peut encore utiliser d'autres dispositifs pour égaliser et synchroniser les pressions à l'entrée.

La présente invention est prévue plus particulièrement pour les équipements d'anesthésie-réanimation, mais elle peut être utilisée dans beaucoup d'autres industries par exemple l'alimentation des chalumeaux oxyacétylénique ou oxhydrique ou les alimentations des fours à gaz.

REVENDICATIONS

1. Un dispositif de sécurité contre l'inversion des branchements dans les appareillages à mélange de gaz.

caractérisé en ce qu'il comporte. pour chaque gaz alimenté, un dispositif (10.11) de commutation à retard sensible à la densité du gaz réellement alimenté. l'ensemble des dispositifs de commutation contrôlant. dans l'ordre décroissant desdites densités, l'ordre dans lequel se produit une variation de pression dans les différentes canalisations (28-29) de liaison reliant ces dispositifs de commutation à une vanne de commutation (12) qui est ouverte pour assurer les intercommunications sur toutes les canalisations (14-15-16-17) de l'appareil de mélange entre l'alimentation (1-2) et l'utilisation lorsque. et seulement lorsque, la canalisation de liaison correspondant au gaz théoriquement le plus léger est soumise la première à ladite variation de pression, ladite ouverture de la vanne interrompant l'alimentation, à partir d'au moins une (14) des canalisations d'alimentation en gaz de l'appareil de mélange. d'un détecteur de pression (18) commandant une alarme.

2. Un dispositif de sécurité selon la revendication 1, caractérisé en ce que, dans le cas d'un appareillage mélangeant plus de deux gaz. celui-ci comporte en série un nombre de vannes de commutation (12) égal au nombre des gaz mélangés moins un, ces vannes étant commandées à l'ouverture, de l'amont vers l'aval, par la variation de pression dans la canalisation de liaison correspondant théoriquement au gaz ayant la densité la plus faible ou au gaz dont la densité est, dans l'échelle des densités des gaz mélangés, immédiatement supérieure à celle du gaz de commande de la vanne (12) immédiatement en amont.

3. Un dispositif de sécurité selon l'une quelconque des revendications 1 et 2, caractérisé en ce que le dispositif de commutation à retard sensible à la densité du gaz réellement alimenté est le dispositif connu dit "minuterie pneumatique" dans lequel le gaz sous pression (21-23) est introduit, par un ajutage d'étranglement (6-7), dans un volume (8-9), la pression dans ce volume étant appliquée comme signal à une vanne tout ou rien (10-11) qui interrompt l'envoi de la pression d'alimentation (26-27) à la canalisation de liaison (28-29) qu'elle contrôle lorsque ladite pression signal atteint une valeur de seuil.

4. Un dispositif de sécurité selon l'une quelconque des revendications 1 à 3, caractérisé en ce qu'il comporte en amont des dispositifs de commutation à retard (10-11) et sur les canalisations (19-20) les alimentant en gaz sous pression, un dispositif d'égalisation des pressions et de synchronisation de l'envoi des pressions égalisées aux dispositifs de commutation à retard.

5. Un dispositif de sécurité selon la revendication 4, caractérisé en ce que le dispositif d'égalisation des pressions et de synchronisation de leur envoi est constitué par un détendeur pilote (3) et au moins deux détendeurs à contre-pression (4-5), le premier détendeur à contre-pression (4) étant piloté par la pression en sortie du détendeur pilote (3) et le second détendeur à contre-pression (5) et les suivants étant pilotés par la pression à la sortie du détendeur à contre-pression (4) d'ordre immédiatement moins élevé. le détendeur pilote (3) et le dernier détendeur à contre-pression étant alimentés à partir de la même canalisation (20).

6. Un dispositif de sécurité selon l'une quelconque des revendications 1 à 5,

caractérisé en ce que dans le cas de l'alimentation avec deux gaz, la vanne de commutation (12) est une vanne à tiroir à double commande pneumatique (32-33) avec sollicitation à la fermeture par un organe de rappel élastique (31) et les canalisations (28-29) d'arrivée des gaz depuis les dispositifs de commutation (10-11) qui aboutissent aux cylindres de commande de la vanne à tiroir (12) sont indépendantes des canalisations (14-15) d'alimentation en gaz de l'appareillage mélangeur, canalisations dont les intercommunications sont contrôlées par la vanne.

# André LEMONNIER

**0246129**

MEMBRE DE LA COMPAGNIE
NATIONALE DES CONSEILS
EN BREVETS D'INVENTION ET DE
L'ASSOCIATION DES CONSEILS
EN PROPRIÉTÉ INDUSTRIELLE

Administration et Marques :
**Rosette ALBERTI**
Conseil en Marques

INGÉNIEUR DES ARTS ET MANUFACTURES
LICENCIÉ EN DROIT

CONSEIL EN BREVETS D'INVENTION

Mandataire agréé auprès de
l'Office Européen des Brevets

4, Boulevard Saint-Denis - 75010 Paris

BREVETS D'INVENTION

Marques de Fabrique
Dessins et Modèles
France et Étranger

Assistance Technique et Juridique
pour les procès en contrefaçon

Consultations en matière
de Propriété Industrielle

Rédaction d'actes de Cession
et de Licence

Tél. (1) 46-07-38-61 & (1) 42-06-68-09
Telex : 216653 F LEMPAT
Câble : LEMPATENT PARIS 128
C.C.P. Paris 7457-44

RECOMMANDE

ACCUSE DE RECEPTION

Paris, le 23 Juin 1987

AL/RL

OFFICE EUROPEEN DES BREVETS
P.B. 5818 Patentlaan 2
2280 HV RIJSWIJK (ZH) Pays Bas

A l'attention de Mr LM GELLIN

Messieurs,

Demande de brevet européen N° 87 400 909 5
au nom de Yves-Paul CORFA

Je reçois votre communication du 18 Juin 1987.

Je vous prie de trouver sous ce pli, pour les joindre au dossier, trois exemplaires de la page 3 que mes Services ont omis, lors de la pagination du texte.

Il s'agit là d'une erreur matérielle évidente dont la correction devrait être autorisée, conformément à la Règle 88, parce que le premier paragraphe de la page 3 est purement et simplement la reproduction de la revendication 1. Le deuxième paragraphe est une explication du fonctionnement de l'invention telle que revendiquée et se retrouve dans la description du mode de réalisation donné à titre d'exemple de la page 8, ligne 22, à la page 9, ligne 21.

./.

MEMBRE D'UNE ASSOCIATION AGRÉEE   LE RÉGLEMENT DES HONORAIRES PAR CHÈQUE EST ACCEPTÉ

Bureau de Loire Atlantique - San Luis, Place du Marché B. P. 2 - 44770 PREFAILLES 40.21.63.96

- 2 -                           **0246129**

La demande dont la priorité est revendiquée ayant été remaniée (le dépôt français ayant été fait directement par l'inventeur), la décision J.04/85 n'est pas applicable.

Vous en remerciant par avance, je vous prie de croire, Messieurs, à l'assurance de mes distingués sentiments.

Page 3 en trois exemplaires.

0246129

**Office européen des brevets**

## RAPPORT DE RECHERCHE EUROPEENNE

Numéro de la demande

EP 87 40 0909

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| A | GB-A-2 041 225 (THE MEDISHIELD CORP. LTD) * Page 1, lignes 39-121; page 2, lignes 19-69,97-112,116-127; figure 1; revendications 1,4 * | 1 | G 01 N 9/32 A 61 M 16/01 |
| | --- | | |
| A | FR-A-2 561 927 (INGENIERIE TECHNIQUE ATLANTIQUE) * Page 1, lignes 4-20; page 2, lignes 6-20; page 2, ligne 34 - page 3, ligne 13; page 3, lignes 28-39; page 4, ligne 4 - page 5, ligne 8; revendications 1,2 * | 1,3 | |
| | --- | | |
| A | US-A-4 487 155 (RUSSELL OLESEN) * Colonne 3, lignes 25-65; colonne 4, lignes 11-21,37-65; figure 1; revendication 1 * | 3 | DOMAINES TECHNIQUES RECHERCHES (Int Cl 4) |
| | --- | | |
| A,D | GB-A-2 001 249 (R.G. HOWISON) * Page 1, ligne 85 - page 2, ligne 13; page 2, lignes 49-69; page 3, lignes 38-64; figure 1; revendications 1,7,17,18 * | 1 | A 61 M 16/00 A 61 M 17/00 G 01 N 9/00 |
| | ----- | | |

Le présent rapport de recherche a été établi pour toutes les revendications

| Lieu de la recherche | Date d'achèvement de la recherche | Examinateur |
|---|---|---|
| LA HAYE | 17-07-1987 | FORMBY N.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82